# EUROPEAN PATENT SPECIFICATION

(11) **EP 4 046 563 B1**
(45) Date of publication and mention of the grant of the patent: **24.07.2024**
(21) Application number: 21158605.2
(22) Date of filing: 23.02.2021
(51) Int. Cl.: A61B 1/00, A61B 1/07, A61B 1/055, G02B 23/24, G02B 6/24, G02B 6/36

(54) **ENDOSCOPE SYSTEM HAVING A ROTATABLE SHAFT**
ENDOSKOPSYSTEM MIT EINEM ROTIERBAREN SCHAFT
SYSTÈME D'ENDOSCOPE COMPRENANT UNE TIGE TOURNANT

(43) Date of publication of application: 24.08.2022
(73) Proprietor: JOSHI INNOVATIONS GmbH, 78573 Wurmlingen (DE)
(72) Inventor: Joshi, Shirish, 78573 Wurmlingen (DE); Ketkale, Sourabh, 78532 Tuttlingen (DE)
(74) Representative: Müller Schupfner & Partner Patent- und Rechtsanwaltspartnerschaft mbB (Muc)

(56) References cited:
- WO-A1-2010/037374
- DE-A1- 102008 049 922
- US-A1- 2015 112 141
- US-A1- 2020 154 979
- US-A1- 2020 397 232
- XU MIAOMIAO ET AL: "Methods of optimizing and evaluating geometrical lightguides with microstructure mirrors for augmented reality displays", OPTICS EXPRESS, vol. 27, no. 4, 18 February 2019 (2019-02-18), pages 5523 - 5543, XP055779991, Retrieved from the Internet <URL:https://www.osapublishing.org/DirectPDFAccess/10461705-6CB8-4A89-97474F35C1891BC3_405068/oe-27-4-5523.pdf?da=1&id=405068&seq=0&mobile=no> DOI: 10.1364/OE.27.005538

## Description

The present invention relates to an endoscope.

Endoscopes are instruments for visualizing the interior of an object. In medical applications, an endoscope is used to examine and inspect the interior of the human body including internal organs, anatomical body cavities, and joints. A typical endoscope includes an elongated flexible or rigid insertion tube (the so-called shaft) within which a lens system is provided. At a tip end of the endoscope an optical system is provided. The image of the object being viewed by the optical system in a field of view is transmitted through the shaft for viewing by the user or for reception by a camera. Some endoscopes also have optical cables for illuminating the field of view with light supplied by an external light source.

Some endoscopes are designed to look at an angle to the axis of the shaft instead of in a straight line, such as 30 degree. The angle in which the field of view may be inclined with respect to the axis of the shaft is defined between the axis of the shaft and a so-called direction of view. The direction of view may be a main direction in which the field of view is directed. The area under observation can be significantly increased by rotating the direction of view around the axis of shaft. The direction of view may be varied by a user, e.g. from 0 to 120 degrees with respect to the shaft axis. By combining inclining the direction of view and rotating the direction of view, a large area can be viewed without significant movement of the shaft of the endoscope. This makes the process safer as more movement of the shaft can cause more unintended injuries to critical structures in the body as well as the endoscope itself. Also the endoscope is more likely to obstruct instruments which might be in use at same time.

Existing rigid endoscope technology generally consists of in-line systems where the tip end of the shaft is along the same axis as the eyepiece at the opposite end of the endoscope. For changing the direction of view, the shaft may be rotated with respect to a fixed body for holding the endoscope. However, this rotation is limited by the light transfer system due to fixedly connected optical cables. That is, during usage of the endoscope, there may be situations in which the user needs to further rotate the shaft in a specific direction but is hindered by a limit stop which is due to the internally connected optical fiber. In addition, if the optical fiber is externally connected to the rotatable shaft, there is the problem that the optical fiber is wounded around the shaft if the shaft is turned by 360°. Therefore, the handling of such endoscopes may be cumbersome. WO 2006052769 A1 discloses a rotatable endoscope system having a rotatable insertion tube. The rotatable insertion tube enables the system to overcome ergonomic deficiencies found in previous endoscope systems by allowing the user to move the view field without rotating the entire endoscope. Further rotatable endoscopes are known from DE 10 2008 049 922 A1, US 2020/0154979 A1 US 2020/0397232 and US 2015/112141.

However, the rotation of the shaft is still limited. Therefore it is an object of the present invention to provide an endoscope having an improved operability to assist a user in handling the endoscope.

The present invention solves this problem with an endoscope having the features of claim 1. In particular, by combining a fully rotatable endoscope and an inclinable direction of view, it is possible to seamlessly follow an object (e.g. an endoscopic tool) with minimal movement of the shaft of the endoscope. Thus potential injuries when automated systems are used may be avoided.

The dependent claims define advantageous embodiments.

The endoscope has a fixed coupling member. Further, the endoscope has a rotatable shaft member including a shaft having a shaft axis extending in an axial direction, wherein the shaft has a tip end and a mounting end, wherein the shaft is configured to transmit light from the mounting end to the tip end and to transmit image information from the tip end to the mounting end. The coupling member and the shaft member are connected to each other such that the shaft member is unlimitedly rotatable by 360° or more about the shaft axis relative to the coupling member. Further, the shaft member has a receiver configured to receive energy.

The shaft member is rotatable without limit stop, by at least 360°, preferably without limit, i.e. round and round any number of times. That is, a fully rotatable endoscope is provided. Accordingly, the endoscope may have an increased operability because a user may readily adjust the direction of view defining the field of view independent of the current position of the shaft member relative to the coupling member. Hence, the endoscope may be also readily implemented in an automated system such as in a robotic system. Further, the Endoscope may have fewer parts to be rotated which may facilitate the handling of the endoscope. In addition, the shaft member (i.e. the rotatable element) may have a compact size because no external cables may be radially connected to the shaft member. Thus, the handling of the endoscope may be further improved. Preferably, if the shaft member is rotated unlimitedly (i.e. without stop), objects to be visualized may be tracked more easily by a user in an uninterrupted manner. Further, the endoscope does not need to be moved in order to have an all-around view. Therefore, the space occupied by the endoscope during a surgery may be reduced. That is, compact size of an endoscope system is very important as operating space in surgery is limited. In addition, several instruments may be cluttering the operating field and extra moving cables reduce surgical freedom and therefore surgical results. In particular, the endoscope may be designed to look at an angle to the axis of the shaft (e.g. the angle may be 30° with respect to the shaft). The angle in which the field of view may be inclined with respect to the axis of the shaft is defined between the axis of the shaft and the direction of view. The area under observation may be significantly increased by being able to fully rotate the direction of view around the axis of shaft. In addition, the direction of view may be inclined with respect to the shaft axis e.g. in a range between 0 to 120 degrees. Generally, the endoscope may provide an image of an object to be examined which is hard to access. Accordingly, the endoscope may have an eye piece (e.g. an ocular) in which a user may see the object (i.e. the image of the object). Alternatively or additionally, the endoscope may have an optical sensor (e.g. an image sensor). Then, an image of the object may be depicted on a display device. The optical sensor may be provided at the tip end of the shaft (e.g. a CCD-sensor) or in a housing connected to the coupling member. In case the sensor is provided at the tip end of the shaft, the image information may be electrical signals that are transmitted through the shaft. On the other hand, in case the sensor is provided within the housing, the image information may be light information that are transmitted through the shaft. The optical sensor is provided within a rotatable part (e.g. the shaft member) of the endoscope. The power supply and/or the signal output may be transmitted from and/or to a stationary part (e.g. the coupling member). For example, the power supply may be realized via the receiver (e.g. wirelessly or via brush contacts). The signal transfer from the optical sensor may be realized wirelessly or via brush contacts.

Throughout the following description the following directions may be referred to:
an axial direction may correspond to an elongation direction of the shaft (e.g. the shaft axis may extend in the axial direction), and
a radial direction which may be a direction perpendicular to the axial direction.

The coupling member is configured so as to allow the shaft member to be unlimitedly rotated about the shaft axis (e.g. to perform two revolutions about the shaft axis in one direction). Fixed in this case may mean that the fixed coupling member may be stationary with respect to the shaft member. In other words, the coupling member may be fixedly held by a user or by another supporting means, while the shaft member may be unlimitedly rotated about the shaft axis. The coupling member (also referred to as coupling attachment) may be a coupling mechanism connecting the shaft member and a fixed body (e.g. the housing). The fixed body may include an eye piece and/or a connection an external camera or viewer system to the endoscope. In addition, the fixed body may include a photo sensor. Accordingly, the fixed body may have an image information passage configured to transmit the image information. Further, the fixed body may have a gripping portion at its outer surface such that a user may ergonomically handle the endoscope.

The coupling member may have an adjustment means for adjusting the focus of the endoscope (i.e. of the image of the object). Preferably, the adjustment means is a focusing ring. Accordingly, by rotating the focusing ring relative to the coupling member, the user may adjust the focus point of the image. Therefore, the endoscope may offer the possibility of adjusting the image (which may be based on the image information) to the optimum sharpness, even for spectacle wearers, by means of the focusing ring. Further, an eyepiece may be arranged or arrangeable at the coupling member. In addition, a coupling device may be connected to the coupling member. The coupling device may be a fixed (i.e. stationary) body with respect to the shaft member. An image sensor may be provided within the coupling device or may be attached to the coupling device so as to receive the image information transmitted by the shaft. The coupling member and the coupling device may be formed as an integral element.

The shaft member may comprise the shaft and a mounting base, wherein the shaft may be mounted to the mounting base so as to rotate together with the mounting base about the shaft axis. The mounting base may be formed in a cylindrical shape having a mounting base axial axis which is aligned with the shaft axis. For example, the receiver may be accommodated within the mounting base. The shaft and the mounting base may be formed as an integral element. Further, the shaft member may be axially symmetrical with respect to the shaft axis. Accordingly, the weight distribution is optimally balanced so as to increase the operability of the endoscope.

The shaft may be formed as a hollow rod like element. For example, the shaft may be an elongated and cylindrical body. Further, the shaft may be flexible or rigid or a combination of both. The flexible shaft may allow an advantageous use of the endoscope in Laparoscopy, gastroscopy, colonoscopy, bronchoscopy and arthroscopy. Further, an endoscope having the flexible shaft may also be used as a cardiac catheter. That is, the shaft may follow a contorted passage so as to continuously investigate the passage and/or objects the passage leads to. The image information may be transferred through the shaft via one optical fiber or a plurality of optical fibers.

The rigid shaft may allow a use of the endoscope as a technical borescope and, in the medical field, as an arthroscope and cystoscope, for example. The shaft may include a rod lens system. Within the rigid shaft, image information may be guided through the rod lens system by rod lenses made of quartz glass, for example, and refracted at air lenses between the rod lenses. This very light-intense design(i.e. a design that absorbs only very little light) allows for smaller lens diameters and thus for smaller shaft diameters.

In any case, the shaft may include an image information guide in the form of an image guide or an electrical conductor. The image guide may transmit image information in the form of light information to a sensor provided within the endoscope or to an external sensor. The electrical conductor may transmit digital image information as data generated by an image sensor provided at the tip end of the shaft.

Further, the shaft may include a light guide for guiding light from the mounting end to the tip end. The light may be introduced into the light guide at the mounting base. For example, the light may be received from a light source (e.g. a LED) provided in the mounting base or from another light source. In case the light source is provided within the mounting base, the light source may be energized by the energy received by the receiver. In this case, the receiver may wirelessly receive energy from an external energy source (more details will follow below). Alternatively, the receiver may wirelessly receive light from an external light source. In this case the light may be transmitted to the receiver without a tethered connection (more details will follow below). Therefore, the boundless/unlimited rotation of the shaft member may be possible.

The light may be emitted by the tip end of the shaft so as to illumine the field of view. Accordingly, an examination and/or inspection may be possible even in dark environments.

That is, the shaft may be configured to transmit the light for illumination in one direction along the shaft axis and the image information in the opposite direction along the shaft axis. Accordingly, both illuminating the field of view and acquiring image information of the field of view may be realized by one single shaft. In other words, the shaft may be configured to transmit light in a bidirectional manner. Therefore, the endoscope may be compact.

The tip end may be also referred to as a distal end of the shaft. The tip end may have an opening through which light may enter and exit the interior of the shaft. The opening may define a surface which may be inclined with respect to the shaft axis by more or less than 90°. That is, the surface may be not perpendicular to the shaft axis. Accordingly, the tip end may be open to a side of the shaft at least partly towards the radial direction.

At the opening an optical system (e.g. a mirror system and/or a prism) may be provided for receiving image information of an object to be imaged. The image information may be light rays reflected by the object. Therefore, the tip end of the shaft may be configured to receive image information from a specific direction (i.e. from a field of view) inclined with respect to the shaft axis. Further, the optical system may be configured so as to redirect the image information of the object into the image information guide, that is, along the orientation of the shaft axis. Subsequently, the image information may be transmitted through the shaft (i.e. via the image information guide) along the shaft axis to the mounting end. As a result, by rotating the shaft about the shaft axis, the direction of view (and thus the field of view) may be rotated so as to provide an all-around view about the tip end. In addition, the optical system may be configured to adjust the angle between the direction of view and the shaft axis so as to change the direction of view. Accordingly, the direction of view may be individually adjusted without moving the shaft so as to holistically image objects existing near the tip end. Thereby, the area that may be imaged by the endoscope without transversely and/or longitudinally moving the endoscope may be enlarged.

Moreover, the tip end of the shaft may have at least one illumination opening for emitting light transmitted from the mounting end to the tip end. Preferably, the illumination opening is provided adjacent to the optical system so as to illuminate the field of view. That is, the illumination opening may be preferably directed towards the same region as the optical system. Preferably, the illumination opening is part of the optical system. For example, the tip end may have four illumination openings so as to provide a sufficient illumination of the field of view.

The mounting end of the shaft may be also referred to as a proximal end of the shaft. The mounting end may be the part of the shaft with which the shaft is mounted to the mounting base. The shaft and the mounting base may be an integral element. Thereby, the maintenance of the endoscope may be facilitated (e.g. no retightening of the shaft and the mounting base may be necessary). Alternatively, the shaft may be detachable from the mounting end. Hence, different shafts may be used with the same mounting base. Accordingly, the endoscope may be adapted to different applications by providing different shafts (e.g. different length of shafts or different optical properties). For example, the shaft may be connected to the mounting base by screwing.

For transmitting the light through the shaft via light guides, glass fibers may be used. However, there may be also provided light guides that can conduct the light using a gel as a transport medium. Gel light guides or liquid light guides offer a stronger light output, which is particularly advantageous for illuminating a large field of view and for digital endoscopy. Liquid light guides may be better suited for transmitting UV or IR light than fiber optics. Gel light guides or liquid light guides may be a bit more awkward to use because they are not as flexible than fiber optic light guides.

The image information (e.g. the reflected light rays from the object to be examined) may be guided through the shaft via the image information guide that may be made up of a plurality of (e.g. many thousands of) individual glass fibers, with a diameter of 7 to 10 µm, for example. In this case a resolution of the image may be 3,000 to 42,000 or 75 × 45 to 240 × 180 pixels, depending on the diameter of the image guide. That is, brightness information and color information for one pixel may be transmitted per fiber. Preferably, the image information guide may be centered on the shaft axis. That is, a central axis of the image information guide may be positioned on the shaft axis. The image information guide may be circumferentially surrounded by the light guide.

Alternatively, the image information may be transmitted via an electrical conductor in case an image sensor is provided at the tip end of the shaft. The electrical conductor may be made of metal. The electrical conductor may transmit the digital image information to the mounting end of the shaft or to the mounting base. Within the mounting base an image transmitter may be provided which may be configured to transmit the image information to a processing system. Preferably, the image transmitter is configured to wirelessly transmit the digital image information.

The shaft member and the coupling member are rotatably connected to each other. Preferably, a radial bearing is provided so as to allow the rotation of the shaft member with respect to the coupling member. That is, in any other degree of freedom a movement of the shaft member with respect to the coupling member may be prevented. Therefore, the endoscope may be securely handled by the user. The bearing may be an anti-friction bearing so as to allow a smooth and easy rotation of the shaft member, such as a ball bearing, a cylindrical roller bearing, a needle roller bearing or a tapered roller bearing.

In this case unlimitedly (i.e. continuously) rotatable by 360° or more may mean that the shaft member may be indefinitely rotatable about the shaft axis with respect to the coupling member. In other words, the shaft member may be rotated several revolutions in series in the same direction. That is, there is no stop provided that may hinder the unlimited or continuous rotation of the shaft member with respect to the coupling member. In addition, there may be no connection by optical fibers or electrical wires between the shaft member and the coupling member. Thus, the handling of the endoscope may be significantly facilitated. In addition, an automized handling of the endoscope may be easily implemented.

The receiver may be a device that may receive energy without being tethered (e.g. being mechanically connected) to an energy source. Therefore, no cable or other energy transmitting element may physically reach from the coupling member to the shaft member. Accordingly, the shaft member and the coupling member may be freely rotatable with respect to each other.

The energy transmitted to the receiver may be in the form of electromagnetic energy or waves such as light or lower frequency electromagnetic energy. The transmission of energy to the receiver may be realized by inductive coupling, resonant inductive coupling, capacitive coupling, magnetodynamic coupling, transmission of microwaves or light waves. In case of inductive coupling the receiver may be formed as a wire coil. In case of resonant inductive coupling the receiver may be formed as a tuned wire coil or a lumped-element resonator. In case of capacitive coupling the receiver may be formed as a metal plate electrode. In case of magnetodynamic coupling, the receiver may be formed as a rotating magnet. In case of transmission of microwaves, the receiver may be formed as an antenna comprising a parabolic dish, phased array or a rectennas rotating magnet. In case of light wave transmission, the receiver may be formed as a photocell. In any of the above cases the receiver may receive energy which may be transformed into electrical energy by a circuitry provided within the shaft member. The electrical energy may be supplied to a light source within the shaft member so as to generate light. The light may then be transferred to the light guide provided within the shaft (further details will follow below).

Further, in case of light wave transmission, the receiver may be formed as a laser receiver or a light transmitting surface. Accordingly, the receiver may be configured to directly receive light which may be then guided to the light guide within the shaft. In this case, the receiver may be configured to transmit the light to the light guide provided within the shaft.

Further, a transmitter may be provided which may be configured to transmit one of the above forms of energy to the receiver. For example, such transmitter may be provided within the coupling member.

That is, between the rotatable shaft member and the stationary coupling member a transfer interface may be formed. Via the transfer interface at least energy may be transferred from the coupling member to the shaft member. In some embodiments, energy may be transferred from the coupling member to the shaft member and image information may be transferred from the shaft member to the coupling member. That is, the transfer interface may transfer energy and image information in a bidirectional manner. In any case, there is preferably no mechanical connection in the interface such that the shaft member may be freely rotatable with respect to the coupling member. The transfer interface may be formed by the receiver of the shaft member and by a transmitter provided within the coupling member. Further, the transfer interface may be provided on an extension of the shaft axis. Accordingly, the endoscope may be compact.

In an embodiment, the coupling member has an image information passage configured to transfer the image information from the shaft member in the axial direction. The image information passage of the coupling member may be aligned to the shaft such that the image information may be transmitted from the shaft to the coupling member independent of the rotational position of the shaft. That is, the image information passage may be aligned to the image information guide of the shaft. For example, the image information passage may be positioned on an extension of the shaft axis so as to be symmetrical to said axis. Accordingly, the image information may be appropriately transmitted from the shaft member to the coupling member independent of the position of the shaft member with respect to the coupling member.

Further, the coupling member may include a lens system, preferably a rod lens system, so as to further transmit the image information to an eye piece and/or to an image sensor. Accordingly, the image information may be transmitted without excessively absorbing light (i.e. information).

In an embodiment, the coupling member has a transmitter configured to input the energy to the receiver in the axial direction or radial direction. As mentioned above, the transmitter may be configured so as to correspond the receiver. Further, the receiver and the transmitter may face each other in the axial direction. That is, the transfer interface may define a rotatable surface (at the shaft member) and a stationary surface (at the coupling member) which may be formed so as to be perpendicular to the axial direction. The receiver and the transmitter may have an annular shape. Accordingly, the image information passage may pass through a center of the receiver and the transmitter. In other words, the receiver and the transmitter may circumferentially surround the image information passage. Therefore, the energy (e.g. light, electromagnetic field or waves) may be sufficiently transmitted from the transmitter to the receiver independent of the relative position of the shaft member and the coupling member. At the same time, the image information may be sufficiently transmitted from the shaft member to the coupling member.

In an embodiment, the coupling member has at least one port for receiving light from a light source, and wherein the coupling member is configured to transmit energy in the form of light to the receiver of the shaft member in the axial direction. An optical waveguide may be attached or attachable to the port so as to transfer light to the coupling member. That is, the waveguide which may be an optical fiber, may be connected to the fixed and stationary part (i.e. the coupling member) of the endoscope. Therefore, there is no such problem like winding the waveguide about the shaft when rotating the shaft. Within the coupling member the light may be further transmitted to the receiver. In more detail, the transmitter (stationary part) may send the light via the transfer interface to the receiver (moving part).

The light source may be an external light source (i.e. may be provided within a separate housing). In particular, xenon lamps may be used as a light source. An external light source may exhibit a power of about 80 Watts. Especially, if the image sensor is provided at the tip end of the shaft, a powerful light source may be necessary to sufficiently illuminate the field of view. However, xenon lamps may generate an enormous amount of heat during operation, most of which is caused by the infrared component of the light source spectrum. Therefore, the IR component may be prevented from reaching the tip end of the shaft in order to prevent an excessive heating of the shaft. Therefore, the light source may be adjustable in light intensity and/or cooled by a fan. Further, the infrared radiation may be removed or reduced from the light spectrum by dichroic concave mirrors and/or by heat protection filters in front of the light guide (i.e. before the light enters into the light guide of the shaft). The dichroic concave mirrors and/or the heat protection filters may be provided within the coupling member so as to reduce the IR component. Therefore, the endoscope may deliver an improved visualization of the object to be examined while an excessive heating of the endoscope may be prevented.

In an embodiment, the at least one port is connected to the coupling member such that the light is inputted into the coupling member in a radial direction perpendicular to the axial direction, and wherein the coupling member is configured to redirect the light so as to be transmitted to the receiver in the axial direction, preferably by a partly reflective mirror array or a micro mirror array.

That is, the coupling member may be configured to redirect the light supplied from an external light source and inputted into the coupling member in the radial direction, along an extension of the shaft axis. That is, the light may be redirected only once within the endoscope. Therefore, the light may be directly inputted into the light guide without being redirected several times. The redirection of the light may be done by reflective mirrors (e.g. micro-mirrors) or prism arrays. The micro mirror may be formed by grooves in an optical substrate (e.g. the optical fiber). The micro-mirror grooves may be spaced apart so as to transmit a collimated beam with symmetrical beam transport. Therefore, the redirected light may be aligned such that the light rays may be essentially parallel to each other. Preferably, the light rays may be redirected by the coupling member such that they are parallel to the axial direction.

The redirection of the light may be realized by a distributor. The distributor may be formed of a light-transmissive substrate. The distributor may have at least one inlet connected to the at least one port and one outlet connected to the transmitter of the coupling member. The shape of the outlet of the distributor may correspond to the shape of the transmitter (e.g. may have an annular shape around an extension of the shaft axis). Further, the distributor may have the partly reflective mirrors or prism arrays so as to evenly distribute and transmit the light at the outlet of the distributor. The partly reflective mirrors or prism arrays may be provided so as to extend over the whole cross section of the light transmissive substrate. Alternatively, the distributor may have the micro-mirror arrays so as to evenly distribute and transmit the light at the outlet of the distributor. The micro-mirror arrays may only partly extend over the cross section of the light transmissive substrate. Preferably, the distributor may align the light rays so as to be essentially parallel to the axial direction.

The alignment of incoming and transmitted light rays may reduce stray reflections which may reduce distortions. As a result, the efficiency of light transmission may be increased. Further details of the mirrors (micro-mirror) or prism arrays could be found in the paper of Miaomiao Xu and Hong Hua "Methods of optimizing and evaluating geometrical lightguides with microstructure mirrors for augmented reality displays" Opt. Express 27, 5523-5543 (2019).

In an embodiment, the coupling member has a transmitter, wherein the transmitter of the coupling member and the receiver of the shaft member are each formed as a light transfer surface, wherein the receiver and the transmitter are facing each other in the axial direction and are configured to transfer the light, wherein the receiver and the transmitter preferably have a circular shape.

In another embodiment, the receiver and the transmitter are arranged circumferentially on an extension of the shaft axis and may have a through hole for passing the image information passage through the receiver and the transmitter. In other words, the receiver and the transmitter may be formed as mating surfaces so as to allow a relative rotation between the shaft member and the coupling member. Accordingly, the receiver and the transmitter may have an annular shape.

In a useful embodiment, the transmitter and receiver are made of glass, transparent plastic or glass fiber.

In addition, the transmitter and receiver may be made of acrylic glass. That is, the receiver and the transmitter may be made of a light-transmissive material so as to reduce refraction and/or reflection. As a result, a contactless transmission of light may be possible. Hence, a relative rotation of the shaft member and the coupling member may be readily possible without limitations.

In an embodiment, the transmitter and the receiver are spaced apart from each other in the axial direction so as to form a gap between the receiver and the transmitter, wherein a transparent fluid is provided within the gap. The gap may be between 0.5 µm and 500 µm, preferably between 3 µm and 20 µm.

The gap may allow a relative movement (e.g. a rotation) of the shaft member and the coupling member without the danger of damaging either the receiver or the transmitter or both due an excessive occurrence of friction. At the same time the fluid may prevent that the light rays are excessively refracted due to being transmitted from a solid medium to a gaseous medium. That is, the fluid provided within the gap may essentially maintain the direction of the light rays (e.g. parallel to the axial direction). Preferably, the fluid has a similar refraction index than the transmitter and/or the receiver. Therefore, the endoscope may exhibit an improved durability and a high light transfer efficiency. The fluid may be gas or a high transmission transparent liquid medium. Especially in the latter case, the light could be transmitted from the coupling member to the shaft member without being excessively refracted at the phase change between air and solid medium. Therefore, the efficiency of light transfer may be further increased. Alternatively, a vacuum may be provided within the gap. Accordingly, the shaft member and the coupling member may comprise a sealing element so as to maintain the vacuum within the gap. Alternatively, the shaft member may be in direct contact with the coupling member. In particular, the receiver may be in direct contact with the transmitter. In this case there may be no gap between the shaft member and the coupling member.

In an embodiment, the shaft member includes at least one light source, preferably a LED, that is operated by electric energy received by the receiver. The light source may comprise a plurality of lamps arranged circumferentially around the extension of the shaft axis. The lamps may be arranged within the mounting base of the shaft member. Accordingly, if the shaft member is rotated, the lamps may be also rotated. Therefore, no transmission of light from a non-moving element to a moving element is necessary. The lamps may be connected to the light guide of the shaft by an optical fiber, for example. In addition, in order to transform the energy received by the receiver into electrical energy, the shaft member may include circuitry. In particular, the shaft member may include a rectifying circuit.

The lamps provided within the shaft member may have a power of about 10 Watts. It is to be noted that the internal lamps may have less power than an external light source. However, the light generated by an internal light source does not have to be transmitted through a flexible cable to the endoscope and may thus not be attenuated due to reflection and/or refraction like the light from the external light source. Therefore, the internal lamps may have less power while nevertheless providing sufficient light so as to illuminate the field of view. Hence, the overall system may have an increased efficiency by reducing the losses during light transfer.

In an embodiment, the energy is inputted to the shaft member by a wireless power supply, preferably by inductive coupling, resonant inductive coupling or by magnetodynamic coupling. The power supply may be an external transmitter transmitting energy to the receiver. The external transmitter may be a coil through which an alternating current may be conducted such that the coil creates an oscillating magnetic field by Ampere's law. The receiver may be a coil through which the magnetic field passes tso as to induce an alternating electromotive force (voltage) by Faraday's law of induction, which creates an alternating current in the receiver. Accordingly, the endoscope may have a low weight because the transmitter may not be provided within the endoscope. Therefore, the operability of the endoscope may be increased. The external power supply may be provided near to the site of operation of the endoscope so as to ensure a sufficient power supply to the endoscope. Therefore, the power supply may be provided in a patient bed on which the patient to be examined may be positioned.

In an embodiment, the coupling member has a transmitter, wherein the transmitter of the coupling member is formed as a transmission coil and the receiver of the shaft member is formed as receiving coil, wherein the receiver and the transmitter are facing each other in the axial direction and are configured to transfer the energy. That is, similar to the arrangement of the light transfer surface of another embodiment of the present invention, in this embodiment the receiving coil and the transmitting coil may be arranged so as to face each other in the axial direction. That is, the coils may be arranged circumferentially around an extension of the shaft axis. Therefore, the image information passage may pass through a center of the coils. Further, the receiving coil and the transmitting coil may be arranged close to each other in order to ensure a sufficient energy transmission.

In another embodiment, the energy is inputted to the shaft member via brush contacts between the rotatable shaft member and the fixed coupling member in the form of electrical energy. In this embodiment, the shaft member may have a cylindrical contact portion. The contact portion may be arranged at the mounting base and may extend in the axial direction. The contact portion may be arranged at an opposite side at the mounting base with respect to the shaft. The contact portion may have electrical contacts as the receiver. The electrical contacts may be provided on a circumference surface of the contact portion facing towards the radial direction. The contacts may be arranged circumferentially at the contact portion. The coupling member may have a receptacle for receiving the contact portion. The receptacle may have contacts corresponding to the contacts of the contact portion. The contacts of the receptacle may be biased by a spring such that a contact between the contacts of the receptacle and the contacts of the contact portion may be ensured. Further, the receptacle may have a bearing for rotatably supporting the shaft member via the contact portion.

In an embodiment, the shaft member has different light sources each having a different wavelength. Accordingly, the illumination of the field of view may be improved by combining different wavelengths. For example, light having the UV or IR wavelength may be used to make differences between substance visible. Therefore, the endoscope may allow improved accuracy during surgery.

The coupling member comprises an actuator for rotating the shaft member relative to the coupling member. That is, a gear may be provided so as to rotate the shaft member by an actuator. The power train may extend on an extension of the shaft axis. That is, the image information passage may circumferentially surround the power train. Alternatively, the power train may be positioned outward in the radial direction from the image information passage and may comprise a threaded rod which may be engaged with a corresponding gear at the shaft member. Accordingly, the endoscope may be automatically operated by a robot or by remote control. In addition, the endoscope may be operated in highly accurate manner.

The coupling member comprises a sensor for detecting the position of the shaft member with respect to the coupling member.

Preferably, the relative position may be determined using a contactless sensor system. For example, the position may be determined, by a magnet wheel provided at the shaft member and a hall sensor provided at the coupling member. The position data may be transferred to an automated operation system so as to accurately control the endoscope. Therefore, a region of interest may be accurately reached and visualized by the endoscope. In addition, the information of the relative position of the shaft member with respect to the coupling member may be useful in an automated operation of the endoscope and in remote operation of the endoscope.

In addition the invention is also directed to a method of using the above endoscope. The method may comprise providing the above endoscope and rotating the shaft member more than 360° in one direction.

Accordingly, a region of interest may be holistically investigated by the 360° viewability. Further, the operability of the endoscope may be facilitated because a user may rotate the shaft member without the need to consider a stop or a limit in the rotational movement.

Further, the invention is also directed to a use of one of the above endoscopes as a borescope for a visual inspection of difficult-to-reach objects in a non-medical field. That is, borescopes may be used for visual inspection work where the target area is inaccessible by other means, or where accessibility may require destructive, time consuming and/or expensive dismounting activities. Borescopes may be used in nondestructive testing techniques for recognizing defects or imperfections. Further, borescopes may be used in the visual inspection of aircraft engines, aero-derivative industrial gas turbines, steam turbines, diesel engines, and automotive and truck engines. Gas and steam turbines require particular attention because of safety and maintenance requirements. Borescope inspection of engines can be used to prevent unnecessary maintenance, which can become extremely costly for large turbines. They are also used in manufacturing of machined or cast parts to inspect critical interior surfaces for burrs, surface finish or complete through-holes. In addition, the borescope may be used in forensic applications in law enforcement and building inspection, and in gunsmithing for inspecting the interior bore of a firearm. Alternatively, one of the above endoscopes may be used in the medical field. The endoscope may be used to visually examine and diagnose, or assist in surgery such as an arthroscopy. In particular, the endoscope may be used in a minimal invasive surgery (e.g. as a laparoscope). For example, the endoscope may be used to examine the internal organs like the throat or esophagus. In particular, the endoscope may be used to inspect human or animal organs like the bladder, the kidneys and the bronchus, or to inspect joints, the colon, the abdomen and the pelvis.

According to a further aspect of the present invention there may be provided an endoscope system comprising one of the above-described endoscopes, an external light source for transmitting light to the endoscope and a display device for displaying the image information transmitted by the endoscope.

Individual features of the above defined embodiments may be rearranged or exchanged with other features so as to form new embodiments. All advantages and modification of said features are analogously applicable to the new embodiments.

In the following the present invention will be explained in detail with reference to the enclosed figures. The detailed explanation of embodiments is provided for better understanding.
**Fig. 1** is a schematic illustration of an endoscope according to an embodiment of the present invention.
**Fig. 2** is a schematic illustration of an endoscope according to an embodiment of the present invention.
**Fig. 3** is a schematic illustration of an endoscope according to an embodiment of the present invention.
**Fig. 4** is a schematic illustration of an endoscope according to an embodiment of the present invention.
**Fig. 5** is a schematic illustration of a light guide according to an embodiment of the present invention.

In the following, embodiments of the present invention will be described with regard to the enclosed figures. Similar features present in each embodiment are described only once and the description of similar features applies to each embodiment.

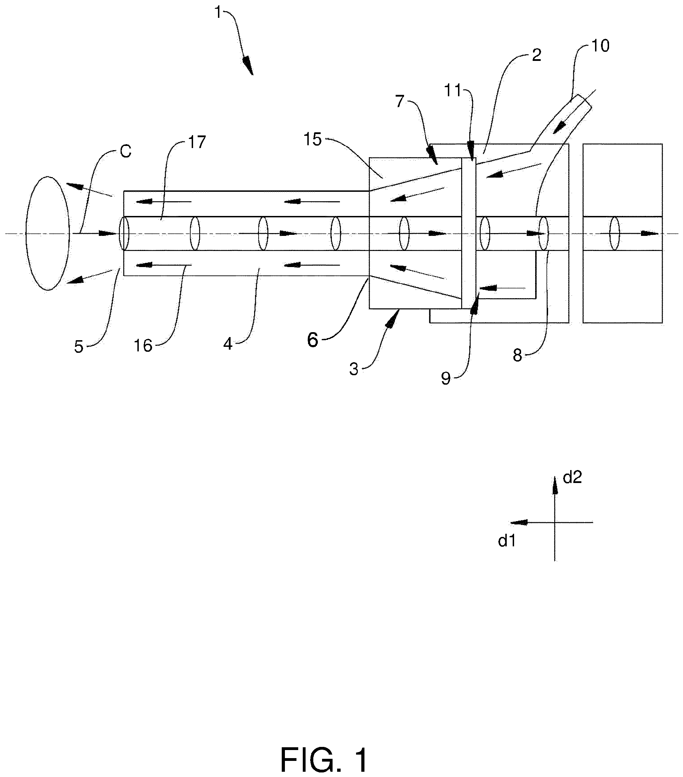

**Fig. 1** is a schematic illustration of an endoscope 1 according to an embodiment of the present invention. In more detail, in Fig. 1 a schematic sectional view along the axial direction is depicted. The endoscope 1 includes a rotatable shaft member 3 and a fixed coupling member 2. The shaft member 3 includes a shaft 4 and a mounting base 15. The shaft 4 has an axial axis C extending along the axial directing d1. Further, the shaft 4 is a hollow member including a light guide 16 and an image guide 17. In particular, the image guide 17 is centered on the shaft axis C and is circumferentially surrounded by the light guide 16. The light guide 16 is configured to transmit light from a mounting end 6 of the shaft member 3 to a tip end 5 of the shaft member 3 (see arrows in the figures). Further, the image guide 17 is configured to transmit image information from the tip end 5 to the mounting end 6 (see arrows in the figures). At the mounting end 6 the shaft 4 is mounted to the mounting base 15.

The shaft member 3 is connected to a coupling member 2 so as to be unlimitedly rotatable with respect to the coupling member by 360° or more. That is, no stop that may limit the shaft member 3 from being infinitely rotated with respect to the coupling member 2 in one direction. The image information is transmitted from the shaft member 3 to the coupling member 2 along the axial direction d1. In particular, the image information is transmitted by the image guide 17 provided within the shaft member 3 and by an image information passage 8 provided within the coupling member 2. In some embodiments the image guide 17 and the image information passage 8 may be similarly configured. In addition, the light is transmitted from the coupling member 2 to the shaft member 3 in the axial direction d1, too, but in the opposite direction as compared to the image information. The light is transmitted by a transmitter 9 of the coupling member 2 to a receiver 7 of the shaft member 3. In the present embodiment the receiver 7 and the transmitter 9 are light transferring surfaces which are arranged opposite to each other sandwiching a gap 11 between them. In other words, the receiver 7 and the transmitter 9 are facing each other in the axial direction d1. The gap 11 is filled with a high transmission transparent liquid so as to reduce friction between the receiver 7 and the transmitter 9 when the shaft member 3 is rotated with respect to the coupling member 2 and to ensure a sufficient light transmission with reduced losses due to refraction. The light transferring surfaces forming the receiver 7 and the transmitter 9 have an annular shape around the extension of the shaft axis C. Further, the extension of the shaft axis C is their center. Accordingly, the image information passage 8 passes through the transmitter 9. In the same way the image guide 17 passes through the receiver 7. As a result, independent of the rotational position of the shaft member 3 and the coupling member 2 to each other, the light transfer from the coupling member 2 to the shaft member 3 and the image information transfer from the shaft member to the coupling member 2 is ensured. In other words, there is no mechanical connection between the shaft member 3 and the coupling member 2 for transferring either light from the coupling member 2 to the shaft member 3 or for transferring image information from the shaft member 3 to the coupling member 2. In some embodiments, the image information is also transmitted via the receiver 7 and the transmitter 9.

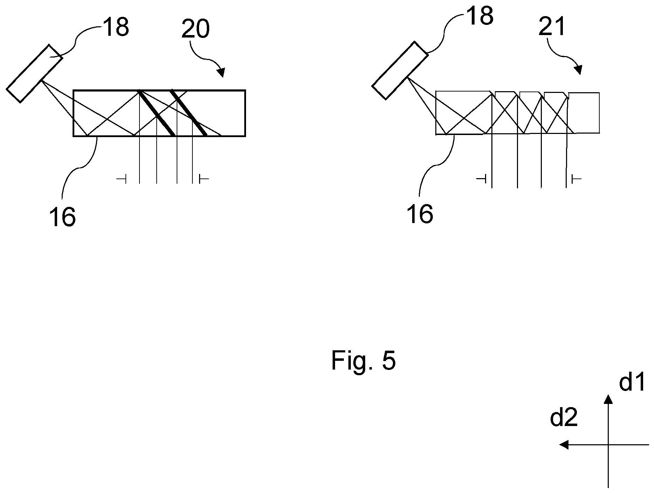

Moreover, the coupling member 2 has a port 10 for being connected with an external light source 18 (not depicted in Fig. 1). Accordingly, light may be inputted to the coupling member 2. The light may be distributed so as to be transferred to the transmitter 9. Further details will be explained with reference to Fig. 5 below.

Further, on the right side of Fig. 1 a coupling device is depicted. The coupling device comprises the image sensor to which the image information may be transmitted. In addition, the coupling device includes ports for connecting further processing devices and/or an eye piece. On the left side of Fig. 1 the object to be imaged is schematically depicted.

In a further embodiment not depicted in the figures, the coupling member 2 may have one or more light sources. That is, the light may be generated in the fixed part (i.e. the coupling member 2) and may be transferred via the transmitter 9 and the receiver 7 to the shaft member 3. In addition, the coupling member 2 may have a cooling arrangement so as to remove heat generated by the light source from the light source.

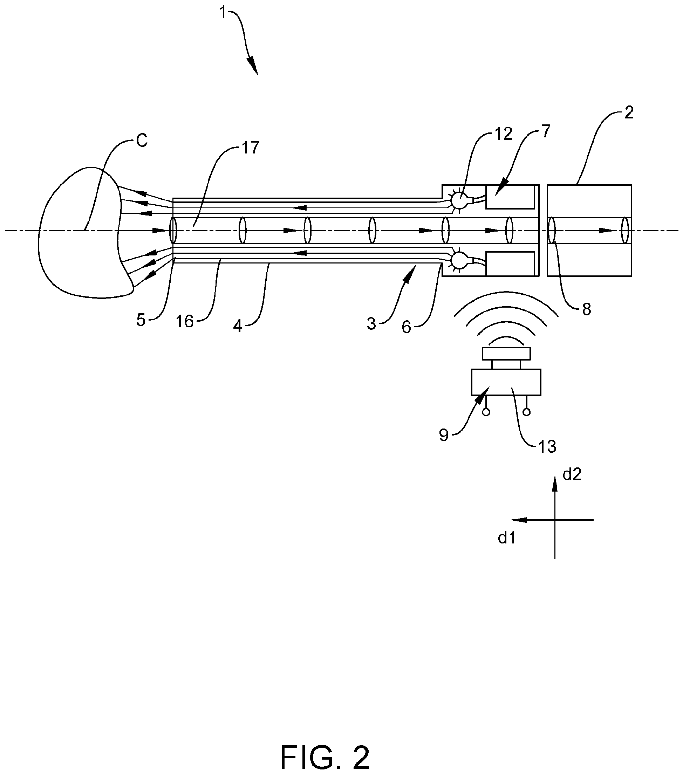

**Fig. 2** is a schematic illustration of an endoscope 1 according to another embodiment of the present invention. The endoscope depicted in Fig. 2 differs from the above embodiment in that there is no light transmission between a rotating part (e.g. the shaft member 3) and a fixed part (e.g. the coupling member 2). In particular, the shaft member 3 comprises lamps 12 which are energized by electrical energy received by the receiver 7 from an external power supply 13. In the present embodiment the external energy supply 13 is an inductive energy transmitter which is positioned near to the endoscope 1 such that the receiver 7 of the shaft member 3 may receive the energy transmitted by the inductive energy transmitter. Within the shaft member 3 a circuitry is provided so as to transform the energy in order to operate the lamps 12. The lamps 12 are connected to the light guide 16 of the shaft 4. The image information is transmitted from the shaft member 3 to the coupling member 2 in a contactless manner. Hence, the shaft member 3 may be readily rotatable with relative to the coupling member 2 in a continuous manner.

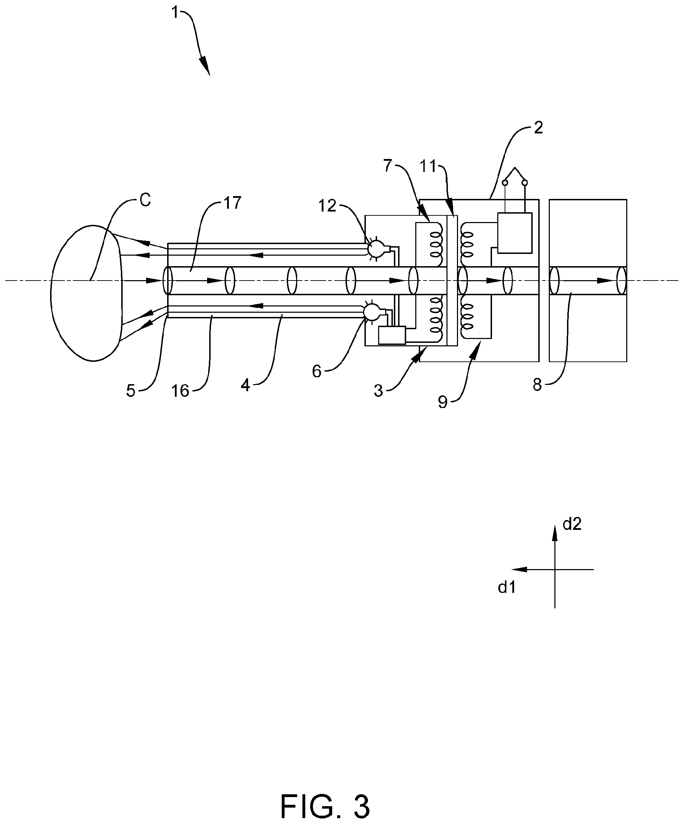

**Fig. 3** is a schematic illustration of an endoscope 1 according to another embodiment of the present invention. The present embodiment differs from the embodiment depicted in Fig. 2 in that the receiver 7 and the transmitter 9 are formed as coils. That is, the receiver 7 is a power reception coil and the transmitter is a transmission coil. Both coils are arranged so as to face each other in the axial direction d1. In particular, the power reception coil and the transmission coil are circumferentially arranged about the extension of the shaft axis C. In the center of the coils the image information passage 8 and the image guide 17 pass through the respective coil. Accordingly, in each relative position of the shaft member 3 and the coupling member 2 to each other, energy may be transmitted to the shaft member 3 and image information may be transmitted to the coupling member 2. Similar as in the above embodiment, the shaft member 3 includes circuitry, in particular a rectifying circuit so as to transform the energy and to operate the lamps 12. In addition, the coupling member 2 includes a connection for power supply in order to supply electrical energy to the transmission coil.

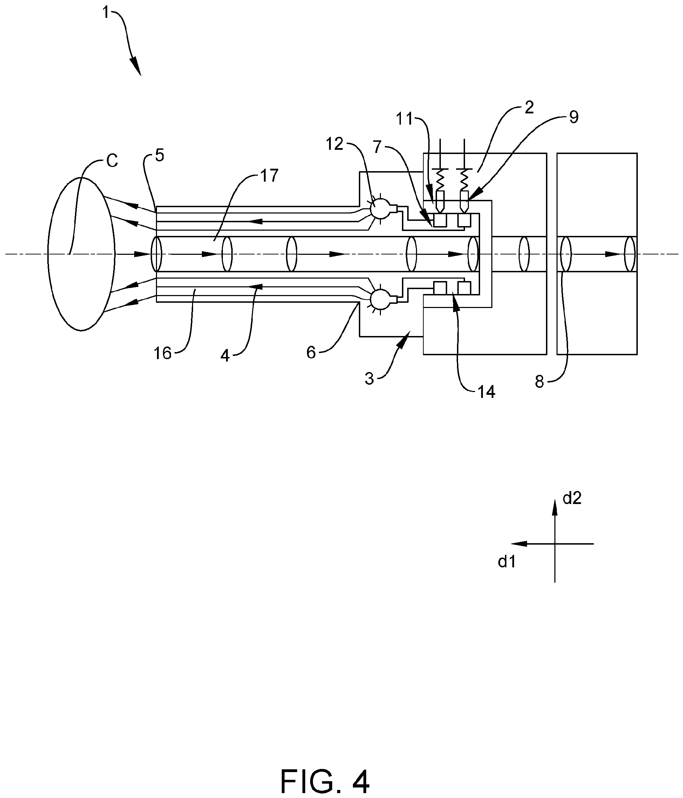

**Fig. 4** is a schematic illustration of an endoscope 1 according to another embodiment of the present invention. The present embodiment has a similar basic structure as the embodiments mentioned before. However, the present embodiment differs from the embodiment depicted in Fig. 3 in that the energy transmission is realized by brush contacts 14. That is, the shaft member 3 may have a contact portion formed as a cylindrical member. The contact portion has circumferential contacts on its outer surface. The circumferential contacts are provided circumferentially about the extension of the shaft axis C. The coupling member 2 has a reception corresponding to the shape of the contact portion of the shaft member 3. The reception is sized such that the contact portion may be accommodated within the receptacle. The receptacle has contacts biased by a spring such that the contacts are pressed against the circumferential contact of the contact portion. Accordingly, the electrical energy may be transmitted from the coupling member 2 to the shaft member 3 without hindering an unlimited rotation of the shaft member 3 relative to the coupling member 2. In a further embodiment the contact portion may be supported by the coupling member 2 via a bearing.

**Fig. 5** is a schematic illustration of a light guide 16 according to an embodiment of the present invention. In particular, in Fig. 5 the part of the light guide 16 or of a distributor is depicted that is configured to redirect the light inputted into the endoscope 1 from an external light source 18. The light from the external light source 18 is inputted into the endoscope via the port 10. Accordingly, the light is inputted in the radial direction d2. Therefore, the light has to be redirected so as to be oriented in the axial direction d1. For this reason the light guide 16 or the distributor may be configured to redirect the light from the radial direction d2 in the axial direction d1 and to evenly distribute the light such that it can be evenly transmitted by the transmitter 9. In this case the light distributor may be arranged within the coupling arrangement 2. In the following, two possibilities will be explained how to realize the redirection of the light. In each case depicted in Fig. 5, the light to be redirected comes from the left side and should be redirected towards the lower side of Fig. 5.

On the left side of Fig. 5 the redirection of the light is realized using a partially reflective mirror array 20. That is, partly reflective mirrors are provided in the cross section of the light guide. Accordingly, a part of the light is reflected in the desired direction (e.g. the axial direction d1). The other part of the light is transferred through the mirror and is then reflected by the next mirror of the mirror array. Accordingly, the redirected light has rays substantially parallel to each other and thus an evenly distributed light intensity.

On the right side of Fig. 5. the redirection of the light is realized using a micro-mirror array 21. That is, mirrors are provided so as to partly extend into the cross section of the light guide. Accordingly, only light is reflected that impinges one of the micro-mirrors is reflected. Similar to the above, the redirected light has rays substantially parallel to each other an evenly distributed light intensity.

In addition, a combination of both above defined redirection methods may be implemented so as to redirect the light. Further, the above-described redirection of light may be implemented for redirecting light that is inputted to the coupling member 2 from an external light source 18 using the distributor. In addition, the above-described redirection of light may be implemented for redirecting light at other positions of the endoscope 1. For example, the above-described redirection may be implemented at the tip end 5 of the shaft 4 so as to direct the light towards the object to be imaged.

### Reference signs

- 1: endoscope
- 2: coupling member
- 3: shaft member
- 4: shaft
- 5: tip end
- 6: mounting end
- 7: receiver
- 8: image information passage
- 9: transmitter
- 10: port
- 11: gap
- 12: lamp
- 13: energy supply
- 14: brush contacts
- 15: mounting base
- 16: light guide
- 17: image guide
- 18: external light source

## Claims

1. Endoscope (1), comprising:
a fixed coupling member (2), and
a rotatable shaft member (3) including a shaft (4) having a shaft axis (C) extending in an axial direction (d1), wherein the shaft (4) has a tip end (5) and a mounting end (6), wherein the shaft (4) is configured to transmit light from the mounting end (6) to the tip end (5) and to transmit image information from the tip end (5) to the mounting end (6),
wherein the shaft member (3) has a receiver (7) configured to receive energy,
wherein the tip end (5) of the shaft (4) is configured to receive the image information from a field of view inclinable with respect to the shaft axis (C), and
wherein the coupling member (2) comprises an actuator for rotating the shaft member (3) relative to the coupling member (2)
**characterised in that**
the coupling member (2) and the shaft member (3) are connected to each other such that the shaft member (3) is indefinitely rotatable about the shaft axis (C) relative to the coupling member (2) and
the coupling member (2) comprises a sensor for detecting the position of the shaft member (3) with respect to the coupling member (2).

2. Endoscope (1) according to claim 1,
wherein the coupling member (2) has an image information passage (8) configured to transfer the image information from the shaft member (3) in the axial direction (d1).

3. Endoscope (1) according to claim 1 or 2, wherein the coupling member (2) has a transmitter (9) configured to input the energy to the receiver (7) in the axial direction (C) or radial direction.

4. Endoscope (1) according to any one of the preceding claims, wherein the coupling member (2) has at least one port (10) for receiving light from a light source, and wherein the coupling member (2) is configured to transmit energy in the form of light to the receiver (7) of the shaft member (3) in the axial direction (C).

5. Endoscope (1) according to claim 4, wherein the at least one port (10) is connected to the coupling member (2) such that the light is inputted into the coupling member (2) in a radial direction (d2) perpendicular to the axial direction (d1), and wherein the coupling member (2) is configured to redirect the light so as to be transmitted to the receiver (7) in the axial direction (d1), preferably by a partly reflective mirror array (20) or a micro mirror array (21).

6. Endoscope (1) according to any one of the preceding claims, wherein the coupling member (2) has a transmitter (9), wherein the transmitter (9) of the coupling member (2) and the receiver (7) of the shaft member (3) are each formed as a light transfer surface, wherein the receiver (7) and the transmitter (9) are facing each other in the axial direction (C) and are configured to transfer the light, wherein the receiver (7) and the transmitter (9) preferably have a circular shape.

7. Endoscope (1) according claim 6, wherein the receiver (7) and the transmitter (9) are arranged circumferentially on an extension of the shaft axis (C) and have a through hole for passing the image information passage through the receiver (7) and the transmitter (9).

8. Endoscope (1) according to claim 6 or 7, wherein the transmitter (7) and receiver (9) are made of glass, transparent plastic or glass fiber.

9. Endoscope (1) according to any one of claims 6 to 8, wherein the transmitter (9) and the receiver (7) are spaced apart from each other in the axial direction (d1) so as to form a gap (11) between the receiver (7) and the transmitter (9), wherein a transparent fluid is provided within the gap (11).

10. Endoscope (1) according to any one of claims 1 to 3, wherein the shaft member (3) includes at least one light source (12), preferably a LED, that is operated by electric energy received by the receiver (7).

11. Endoscope (1) according to any one of claims 1 to 3 or 10, wherein the energy is inputted to the shaft member (3) by a wireless power supply (13), preferably by inductive coupling, resonant inductive coupling or by magnetodynamic coupling.

12. Endoscope (1) according to claim 11, wherein the coupling member (2) has a transmitter (9), wherein the transmitter (9) of the coupling member (2) is formed as a transmission coil and the receiver (7) of the shaft member (3) is formed as receiving coil, wherein the receiver (7) and the transmitter (9) are facing each other in the axial direction (d1) and are configured to transfer the energy.

13. Endoscope according to any one of claims 1 to 3 or 10, wherein the energy is inputted to the shaft member (3) via brush contacts (14) between the rotatable shaft member (3) and the fixed coupling member (2) in the form of electrical energy.

14. Endoscope (1) according to any one of claims 1 to 3 or 10 to 13, wherein the shaft member (3) has different light sources (12) each having a different wavelength.

## Patentansprüche

1. Endoskop (1), umfassend:
ein festes Kopplungselement (2), und
ein drehbares Schaftelement (3) mit einem Schaft (4) mit einer Schaftachse (C), die sich in einer axialen Richtung (d1) erstreckt, wobei der Schaft (4) ein Spitzenende (5) und ein Montageende (6) aufweist, wobei der Schaft (4) so konfiguriert ist, dass sie Licht von dem Montageende (6) zu dem Spitzenende (5) überträgt und Bildinformationen von dem Spitzenende (5) zu dem Montageende (6) überträgt,
wobei das Schaftelement (3) einen Empfänger (7) aufweist, der zur Aufnahme von Energie konfiguriert ist,
wobei das Spitzenende (5) des Schafts (4) so konfiguriert ist, dass es die Bildinformationen aus einem in Bezug auf die Schaftachse (C) neigbaren Sichtfeld empfängt, und
wobei das Kopplungselement (2) einen Aktuator zum Drehen des Schaftelements (3) relativ zum Kopplungselement (2) umfasst, **dadurch gekennzeichnet, dass** das Kopplungselement (2) und das Schaftelement (3) so miteinander verbunden sind, dass das Schaftelement (3) relativ zum Kopplungselement (2) unbegrenzt um die Schaftachse (C) drehbar ist und
das Kopplungselement (2) einen Sensor zur Erfassung der Position des Schaftelements (3) in Bezug auf das Kopplungselement (2) umfasst.

2. Endoskop (1) nach Anspruch 1,
wobei das Kopplungselement (2) einen Bildinformationsdurchgang (8) aufweist, der so konfiguriert ist, dass er die Bildinformationen von dem Schaftelement (3) in der axialen Richtung (d1) überträgt.

3. Endoskop (1) nach Anspruch 1 oder 2, wobei das Kopplungselement (2) einen Sender (9) aufweist, der so konfiguriert ist, dass er die Energie in axialer Richtung (C) oder in radialer Richtung in den Empfänger (7) einspeist.

4. Endoskop (1) nach einem der vorhergehenden Ansprüche, wobei das Kopplungselement (2) mindestens eine Öffnung (10) zur Aufnahme von Licht von einer Lichtquelle aufweist, und wobei das Kopplungselement (2) konfiguriert ist, um Energie in Form von Licht an den Empfänger (7) des Schaftelements (3) in axialer Richtung (C) zu übertragen.

5. Endoskop (1) nach Anspruch 4, wobei die mindestens eine Öffnung (10) mit dem Kopplungselement (2) so verbunden ist, dass das Licht in einer radialen Richtung (d2) senkrecht zur axialen Richtung (d1) in das Kopplungselement (2) eingespeist wird, und wobei das Kopplungselement (2) so konfiguriert ist, dass es das Licht so umleitet, dass es in der axialen Richtung (d1) zum Empfänger (7) übertragen wird, vorzugsweise durch eine teilreflektierende Spiegelanordnung (20) oder eine Mikrospiegelanordnung (21).

6. Endoskop (1) nach einem der vorhergehenden Ansprüche, wobei das Kopplungselement (2) einen Sender (9) aufweist, wobei der Sender (9) des Kopplungselements (2) und der Empfänger (7) des Schaftelements (3) jeweils als Lichtübertragungsfläche ausgebildet sind, wobei der Empfänger (7) und der Sender (9) in axialer Richtung (C) einander zugewandt und zur Übertragung des Lichts konfiguriert sind, wobei der Empfänger (7) und der Sender (9) vorzugsweise eine kreisförmige Form aufweisen.

7. Endoskop (1) nach Anspruch 6, wobei der Empfänger (7) und der Sender (9) in Umfangsrichtung auf einer Verlängerung der Schaftachse (C) angeordnet sind und ein Durchgangsloch zum Durchführen des Bildinformationsdurchgangs durch den Empfänger (7) und den Sender (9) aufweisen.

8. Endoskop (1) nach Anspruch 6 oder 7, wobei der Sender (7) und der Empfänger (9) aus Glas, transparentem Kunststoff oder Glasfaser hergestellt sind.

9. Endoskop (1) nach einem der Ansprüche 6 bis 8, wobei der Sender (9) und der Empfänger (7) in axialer Richtung (d1) voneinander beabstandet sind, so dass ein Spalt (11) zwischen dem Empfänger (7) und dem Sender (9) gebildet wird, wobei in dem Spalt (11) eine transparente Flüssigkeit vorgesehen ist.

10. Endoskop (1) nach einem der Ansprüche 1 bis 3, wobei das Schaftelement (3) mindestens eine Lichtquelle (12), vorzugsweise eine LED, aufweist, die durch die vom Empfänger (7) empfangene elektrische Energie betrieben wird.

11. Endoskop (1) nach einem der Ansprüche 1 bis 3 oder 10, wobei die Energie über eine drahtlose Stromversorgung (13), vorzugsweise durch induktive Kopplung, resonante induktive Kopplung oder durch magnetodynamische Kopplung, in das Schaftelement (3) eingespeist wird.

12. Endoskop (1) nach Anspruch 11, wobei das Kopplungselement (2) einen Sender (9) aufweist, wobei der Sender (9) des Kopplungselements (2) als Sendespule und der Empfänger (7) des Schaftelements (3) als Empfangsspule ausgebildet ist, wobei der Empfänger (7) und der Sender (9) in axialer Richtung (d1) einander zugewandt und zur Übertragung der Energie konfiguriert sind.

13. Endoskop nach einem der Ansprüche 1 bis 3 oder 10, wobei die Energie über Bürstenkontakte (14) zwischen dem drehbaren Schaftelement (3) und dem festen Kopplungselement (2) in Form von elektrischer Energie in das Schaftelement (3) eingespeist wird.

14. Endoskop (1) nach einem der Ansprüche 1 bis 3 oder 10 bis 13, wobei das Schaftelement (3) verschiedene Lichtquellen (12) mit jeweils unterschiedlichen Wellenlängen aufweist.

## Revendications

1. Endoscope (1), comprenant :
un élément de couplage fixe (2), et
un élément formant arbre rotatif (3) incluant un arbre (4) ayant un axe d'arbre (C) s'étendant dans une direction axiale (d1), dans lequel l'arbre (4) a une extrémité de pointe (5) et une extrémité de montage (6), dans lequel l'arbre (4) est configuré pour transmettre une lumière depuis l'extrémité de montage (6) jusqu'à l'extrémité de pointe (5) et pour transmettre des informations d'image depuis l'extrémité de pointe jusqu'à l'extrémité de montage (6),
dans lequel l'élément formant arbre (3) a un récepteur (7) configuré pour recevoir de l'énergie,
dans lequel l'extrémité de pointe (5) de l'arbre (4) est configurée pour recevoir les informations d'image depuis un champ de vision pouvant être incliné par rapport à l'axe d'arbre (C), et
dans lequel l'élément de couplage (2) comprend un actionneur destiné à mettre en rotation l'élément formant arbre (3) relativement à l'élément de couplage (2),
**caractérisé en ce que**
l'élément de couplage (2) et l'élément formant arbre (3) sont connectés l'un à l'autre de telle sorte que l'élément formant arbre (3) peut être indéfiniment mis en rotation autour de l'axe d'arbre (C) relativement à l'élément de couplage (2) et
l'élément de couplage (2) comprend un capteur destiné à détecter la position de l'élément formant arbre (3) par rapport à l'élément de couplage (2).

2. Endoscope (1) selon la revendication 1,
dans lequel l'élément de couplage (2) a un passage d'informations d'image (8) configuré pour transmettre les informations d'image provenant de l'élément formant arbre (3) dans la direction axiale (d1).

3. Endoscope (1) selon la revendication 1 ou 2, dans lequel l'élément de couplage (2) a un émetteur (9) configuré pour entrer l'énergie vers le récepteur (7) dans la direction axiale (C) ou dans la direction radiale.

4. Endoscope (1) selon l'une quelconque des revendications précédentes, dans lequel l'élément de couplage (2) a au moins un orifice (10) destiné à recevoir une lumière provenant d'une source de lumière, et dans lequel l'élément de couplage (2) est configuré pour émettre une énergie sous forme de lumière vers le récepteur (7) de l'élément formant arbre (3) dans la direction axiale (C).

5. Endoscope (1) selon la revendication 4, dans lequel ledit au moins un orifice (10) est connecté à l'élément de couplage (2) de telle sorte que la lumière est entrée jusque dans l'élément de couplage (2) dans une direction radiale (d2) perpendiculaire à la direction axiale (d1), et dans lequel l'élément de couplage (2) est configuré pour rediriger la lumière afin qu'elle soit transmise jusqu'au récepteur (7) dans la direction axiale (d1), de préférence via un réseau de miroirs partiellement réfléchissants (20) ou un réseau de micro-miroirs (21).

6. Endoscope (1) selon l'une quelconque des revendications précédentes, dans lequel l'élément de couplage (2) a un émetteur (9), dans lequel l'émetteur (9) de l'élément de coupage (2) et le récepteur (7) de l'élément formant arbre (3) sont chacun formés en tant que surface de transfert de lumière, dans lequel le récepteur (7) et l'émetteur (9) sont en face l'un de l'autre dans la direction axiale (C) et sont configurés pour transférer la lumière, dans lequel le récepteur (7) et l'émetteur (9) ont de préférence une forme circulaire.

7. Endoscope (1) selon la revendication 6, dans lequel le récepteur (7) et l'émetteur (9) sont agencés de manière circonférentielle sur une extension de l'axe d'arbre (C) et un trou traversant destiné à faire passer le passage d'informations d'image à travers le récepteur (7) et l'émetteur (9).

8. Endoscope (1) selon la revendication 6 ou 7, dans lequel le récepteur (7) et l'émetteur (9) sont faits à partir de verre, de plastique transparent ou de fibres de verre.

9. Endoscope (1) selon l'une quelconque des revendications 6 à 8, dans lequel l'émetteur (9) et le récepteur (7) sont à distance l'un de l'autre dans la direction axiale (d1) de manière à former un intervalle (11) entre lequel le récepteur (7) et l'émetteur (9), dans lequel un liquide transparent est prévu à l'intérieur de l'intervalle (11).

10. Endoscope (1) selon l'une quelconque des revendications 1 à 3, dans lequel l'élément formant arbre (3) inclut au moins une source de lumière (12), de préférence une LED, qui peut fonctionner via une énergie électrique reçue par le récepteur (7).

11. Endoscope (1) selon l'une quelconque des revendications 1 à 3 ou 10, dans lequel l'énergie est entrée vers l'élément formant arbre (3) via une alimentation de puissance sans fil (13), de préférence par couplage inductif, par couplage inductif à résonance, ou par couplage magnéto-dynamique.

12. Endoscope (1) selon la revendication 11, dans lequel l'élément de couplage (2) a un émetteur (9), dans lequel l'émetteur (9) de l'élément de couplage (2) est formé en tant que bobine de transmission et le récepteur (7) de l'élément formant arbre (3) est formé en tant que bobine de réception, dans lequel le récepteur (7) et l'émetteur (9) sont en face l'un de l'autre dans la direction axiale (d1) et sont configurés pour transmettre l'énergie.

13. Endoscope (1) selon l'une quelconque des revendications 1 à 3 ou 10, dans lequel l'énergie est entrée vers l'élément formant arbre (3) via des contacts balais (14) entre l'élément formant arbre rotatif (3) et l'élément de couplage fixe (2) sous forme d'énergie électrique.

14. Endoscope (1) selon l'une quelconque des revendications 1 à 3 ou 10 à 13, dans lequel l'élément formant arbre (3) a des sources de lumière différentes (12) ayant chacune une longueur d'onde différente.
